# EUROPÄISCHE PATENTANMELDUNG

(11) **EP 1 095 660 A2**
(43) Veröffentlichungstag der Anmeldung: **02.05.2001**
(21) Anmeldenummer: 00122947.5
(22) Anmeldetag: 21.10.2000
(51) Int. Cl.: A61K 35/78, A61K 31/78

(54) **Therapeutisch wirksame Kombination welche Terpinen-4-ol und Arzneipflanzen enthalt**

(30) Priorität: 27.10.1999 CH 196099
(71) Anmelder: Bogar AG, 8002 Zürich (CH)
(72) Erfinder: Frater-Schröder, Marijke, Dr.phil.II, 8400 Winterthur (CH); Brühwiler, Klemens, Dr.med., 8353 Elgg (CH)
(74) Vertreter: Patentanwälte Schaad, Balass, Menzl & Partner AG

(57) **Zusammenfassung**

Es wird eine therapeutisch wirksame Kombination enthaltend 5-25 Gew.% von Terpinen-4-ol enthaltenden ätherischen Ölen und 0.01 bis 10 Gew.% von Gerbstoffe enthaltenden Arzneipflanzen bzw. deren Extrakten, welche zur topischen Behandlung von Haut- und Schleimhaut Affektionen im Tiergesundheitsbereich verwendet wird.

Als Terpinen-4-ol enthaltendes ätherisches Öl verwendet man insbesondere Teebaumöl und als Gerbstoffe enthaltende Arzneipflanze bzw. deren Extrakt insbesondere einen Ratanhiawurzel-Extrakt.

## Beschreibung

Die Erfindung betrifft eine therapeutisch wirksame Kombination enthaltend 5-25 Gew.% von Terpinen-4-ol enthaltenden ätherischen Ölen und 0.01 bis 10 Gew.% von Gerbstoffe enthaltenden Arzneipflanzen bzw. deren Extrakten oder Tinkturen, welche zur topischen Behandlung von Haut- und Schleimhaut Affektionen im Tiergesundheitsbereich verwendet wird.

Als Terpinen-4-ol enthaltende ätherische Öle verwendet man erfindungsgemäss insbesondere Teebaumöl. Ebenfalls möglich ist die Verwendung von Majoranöl und Marjolain Essenz. Als Gerbstoffe enthaltende Arzneipflanzen verwendet man beispielsweise Ratanhiawurzel (Ratanhiae radix), insbesondere einen Ratanhiawurzel-Extrakt. Besonders bevorzugte Arzneipflanzen sind Uvae ursi folium, Quercus cortex, Thea nigra, Tormentillae radix, Hamamelidis folium, Ratanhiae radix, Hamamelidis cortex, Orthosiphonis folium und Juglandis folium. Teebaumöl (Melaleucae aetheroleum) ist das durch Wasserdampfdestillation aus den Blättern und Zweigspitzen von Melaleuca alternifolia Cheel, Melaleuca linariifolia Sm., Melaleuca dissitiflora Mueller oder anderen Arten der Gattung Melaleuca (Myrtaceae) gewonnene ätherische Öl.

Die erfindungsgemässe synergistische Kombination enthält 5-25 Gew.%, vorzugsweise 10 Gew.% von Terpinen-4-ol enthaltenden ätherischen Ölen, und 0.01 bis 10 Gew.%, vorzugsweise 0.1 bis 2 Gew.%, von Gerbstoff enthaltenden Arzneipflanzen bzw. deren Extrakte oder Tinkturen, wobei die Extrakte bzw. die Tinkturen vorzugsweise 0.1-2 Gew.% der Droge enthalten. Besonders bevorzugt ist Ratanhiawurzel-Extrakt. Ratanhiawurzel-Extrakt enthält nicht weniger als 5 Gew.% Gerbstoffe, ausgedrückt als Pyrogallol. In diesen Dosierungen zeigt die erfindungsgemässe Kombination keine toxischen Reaktionen.

Das in der erfindungsgemässen Kombination insbesondere als Bestandteil verwendete Teebaumöl ist bekannt (siehe beispielsweise DE-OS 196 23 905) und dessen therapeutische Wirkung wird in zahlreichen Literaturstellen beschrieben. Es enthält neben mindestens 30 Gew.% Terpinen-4-ol noch höchstens 15 Gew.% 1,8-Cineol und ferner auch noch Cymol, Pinen, Terpineol, α-Terpinen, Limonen, α-Terpineol, γ-Terpinen. Die ferner noch in der erfindungsgemässen Kombination insbesondere als Bestandteile verwendeten Ratanhiawurzel-Extrakte sind ebenfalls bekannt und deren therapeutische Wirkung wird in zahlreichen Literaturstellen beschrieben.

In EP 0 870 507 A1 wird gezeigt, dass Zubereitungen, die 0.02 bis 5 Gew.% eines ätherischen Öls sowie 1 bis 10 Gew.% eines Kräuterextraktes enthalten, dessen Gehalt nicht definiert ist, in vitro eine synergistische antimikrobielle Wirkung haben, wobei mit Ausnahme des Mundes, kein spezifisches Zielorgan angegeben wurde. Unter anderem wurde Teebaumöl als ätherisches Öl in der Zubereitung verwendet. In WO 99/34811 wird von den gleichen Erfindern gezeigt, dass Zubereitungen, die 0.1 bis 5 Gew.% eines ätherischen Öls sowie 1 bis 10 Gew.% eines Kräuterextraktes enthalten, dessen Gehalt nicht definiert ist, in vitro nur eine antimykotische Wirkung aufweisen. Dabei wurden teilweise Zubereitungen verwendet, die eine identische Zusammensetzung wie diejenigen Zubereitungen aufweisen, die in 0 870 507 A1 beschrieben sind. In der neueren Publikation (WO 99/34811) konnte höchstens additive, eher eine nur auf das ätherische Öl beschränkte Wirkung, nicht jedoch eine synergistische Wirkung gezeigt werden. Ein grundlegendes Problem bei der Anwendung antimykotischer Zubereitungen ist ihre mangelnde Wirksamkeit bei Mischinfektionen, was sich nachteilig auswirkt, da die meisten Infektionen Mischinfektionen sind.

Aus der Deutschen Apothekerzeitung, 1995, 135, 40-48 ist bekannt, dass Teebaumöl in dermatologischen und stomatologischen Präparaten, beispielsweise Shampoos, Hand und Körpercremes und Lotionen angewendet wird. Im Journal of Family Practice, Vol. 38, No. 6 (Jun), 1994 wird die in vivo Behandlung von Patienten, die an Onychomycose erkrankt sind, mit 100% Teebaumöl beschrieben. Dabei konnte nur ein mässiger Erfolg erzielt werden, insbesondere wurde eine hohe Rekurrensrate verzeichnet. Bei einem in vivo Vergleich von 10 Gew.% Teebaumöl-Creme mit 1 Gew.% Tolnaftat bei der Behandlung von Patienten mit durch Tinea Pedis verursachten Manifestationen hat sich aber gezeigt, dass Teebaumöl allein eine sehr schwache oder keine antimykotische Wirkung besitzt, die mit derjenigen von Placebo gleichzusetzen ist (siehe hierzu Austral. J. Dermatol. 1992; 33: 145-149). In einer neuen in vivo Studie, die in Tropical Medicine and International Health (1999; 4; 284-287) beschrieben ist, wurde ein Vergleich von einer 5 Gew. % Teebaumöl enthaltenden Creme mit einer Creme, die 5 Gew.% Teebaumöl und 2% Butenafinhydrochloride enthielt, bei der Behandlung von an Onychomycose leidenden Patienten ausgeführt. Dabei konnte bei der Behandlung mit nur teebaumölhaltiger Creme kein Effekt erzielt werden.

Zusammenfassend zeigt die neuere Literatur, dass die Resultate der bisher mit teebaumölhaltigen Zubereitungen ausgeführten, klinischen Studien in vivo nicht so überzeugend sind wie die in vitro Experimente, die in der älteren Literatur beschrieben sind (siehe zum Beispiel Forsch Komplementärmed 2000, 7:17-20).

Bei der Verwendung teebaumölhaltiger Hautpflege- und Kosmetikprodukten werden ausserdem häufig Hautirritationen und allergische Reaktionen beobachtet. In Pharmazie, 1998, 143, 26-30 wurde gezeigt, dass diese Hautirritationen und allergischen Reaktionen in erster Linie auf Oxidationsprodukte zurückzuführen sind, die durch Autooxidation bei der Lagerung des ätherischen Öls entstehen.

Was den Gerbstoffe haltigen Ratanhiawurzel-Extrakt anbelangt, so war u.a. bekannt (siehe beispielsweise Deutsche Apotheker Zeitung 127 Jahrg. Nr. 44, Seiten 2256-2258 vom 29.10.1987), dass dieser aufgrund seiner adstrigierenden Wirkung in der Lage ist die Durchlässigkeit der Gewebe zu vermindern, die mechanische Widerstandsfähigkeit der Gewebe zu steigern, die Resorbierbarkeit toxischer Stoffe zu verhindern, kapillare Blutungen zu stillen und einen milden antimikrobiellen Effekt zu erzielen. Ferner war es auch bekannt Ratanhiawurzel-Extrakte bei bakteriellen Erkrankungen und Pilzerkrankungen der Haut einzusetzen (siehe beispielsweise die DE-OS 364 04 09 von Sato Pharm Co.). Weiterhin war bekannt, dass die Gerbstoffdrogen eine antioxidative Wirkung aufzeigen und Radikalfängereigenschaften besitzen.

Erfindungsgemäss wurde nunmehr gefunden, dass eine therapeutisch wirksame wässrige Kombination, enthaltend 5 bis 25 Gew.% von Terpinen-4-ol enthaltenden ätherischen Ölen und 0.01 - 10 Gew.% von Gerbstoffe enthaltenden Arzneipflanzen bzw. deren Extrakten, überraschenderweise in einer klinischen Studie gezeigt werden, dass im Bereich der chronischen Dermatitiden bei Tieren eine synergistische Wirkung bei der in vivo Behandlung von Haut- und Schleimhaut Affektionen, d.h. Mischinfektionen, erzielt wird, die bei topischer Anwendung im Tiergesundheits Bereich eine ausserordentlich gute Heilwirkung ergibt. Aufgrund dieser synergistischen Wirkung erweist sich die erfindungsgemässe Kombination bei Haut- und Schleimhaut Affektionen als antimykotisch, antibakteriell, entzündungshemmend und antiviral wirksam und sie kann überdies zur Wundheilung eingesetzt werden. Sie ist ebenfalls wirksam bei unspezifischem Ekzem, bei allergischer Dermatitis, bei Flohbissallergie, bei der Zwischenzehenpyodermie, Leckdermatitis und der Hautfaltenpyodermie von Hund und Katze. Aufgrund dieser Wirkungen kann die erfindungsgemässe Kombination u.a. zur Behandlung von Krankheitssymptomen wie zum Beispiel Juckreiz (Pruritus), der Rötung (Erythem), Erosion, von Papeln, Pusteln, nässender Oberfläche, Eiter, Krusten, Haarausfall (Alopezie), Hautverdickung, Urtikaria, Abschuppung, Hyperpigmentation und Vesikel, aber auch von Schnitt- und Schürfwunden, Virusinfektionen, Bakterien-und Pilzinfektionen, von Infektionen im Urogenitalbereich, Geschwüren und Furunkeln, ferner aber auch von Allergien und anderen Hautproblemen, beispielsweise denjenigen, welche durch Überzüchtung entstehen, eingesetzt werden.

Überraschenderweise erstreckt sich die synergistische therapeutische Wirkung der erfindungsgemässen Kombination insbesondere auch auf Mischinfektionen der vorgenannten Art. Damit stellt die erfindungsgemässe neue, therapeutisch wirksame Kombination eine überraschend wirksame willkommene Alternative zu der bisher üblichen Antibiotika und/oder Cortison Therapien dar.

Die in vivo überraschend gute Wirkung der erfindungsgemässen Kombination ergibt sich vermutlich auch daraus, dass beispielsweise Mikroorganismen durch die Bestandteile der Kombination von mindestens zwei Angriffspunkten her angegriffen und abgetötet werden, wobei die Wirkung überadditiv d.h. synergistisch eintritt. Ebenfalls überraschend ist die antimykotische Wirkung der Kombination in vivo, die aufgrund der oben besprochenen praktischen Unwirksamkeit von Teebaumöl allein in vivo bei der Behandlung von Tinea Pedis nicht zu erwarten war. Die überraschend gute antivirale Wirkung der erfindungsgemässen Kombination geht vermutlich einher mit einer Verklumpung und damit einer Inaktivierung von Virenpartikeln.

Die tiertherapeutische Anwendung kann bei einer Vielzahl von Tiergattungen mit ähnlicher Hautbeschaffenheit erfolgen. Hierbei sind insbesondere Säugetiere, wie Hunde, Katzen und Pferde (sog.Companion Animals), Federtiere, wie Vögel und Hühner, Nutztiere, wie Kühe, Schweine und Kaninchen hervorzuheben. Besonders wichtig sind dabei behaarte Tiere.

Die erfindungsgemässe Kombination zeigt auch eine gute Wirkung bei der topischen Behandlung von Haut- und Schleimhaut Affektionen beim Menschen.

Die Herstellung von Terpinen-4-ol enthaltenden Zubereitungen, die eine hohe Lagerstabilität aufweisen ist ausgesprochen anspruchsvoll. Mit der erfindungsgemässen Zubereitung ist nicht nur gelungen durch die Zugabe eines gerbstoffhaltigen Kräuterextraktes eine synergistische Wirkung zu erzielen. Es hat sich auch herausgestellt, dass beispielsweise der Ratanhiaextrakt die Autooxidation von Terpinen-4-ol inhibiert. Dadurch hat die erfindungsgemässe Zubereitung eine ausgesprochen hohe Lagerstabilität und ist mithin auch sehr gut verträglich.

In einer bevorzugten Ausführungsform weist die erfindungsgemässe Zubereitung einen hohen Wassergehalt auf. Zur Stabilisierung einer solchen Emulsion mit hohem ätherischen Öl Gehalt über einen längeren Zeitraum wird ein Hilfsmittel benötigt, da sonst eine Entmischung der Öl- und Wasserphase auftritt. Bei der erfindungsgemässen Zubereitung wird durch Zugabe von 5-Ureidohydantoin einer solchen Entmischung vorgebeugt. Die topische Anwendung der erfindungsgemässen Kombination kann in allen möglichen topischen Anwendungsformen, beispielsweise als Emulsionen, Cremen, Salben, Gelen oder Flüssigkeiten, wie zum Beispiel Ölen oder Tinkturen, erfolgen, die neben den oben angeführten Bestandteilen der erfindungsgemässen Kombination noch die in galenischen Zubereitungen üblichen Zusätze enthalten.

Das nachfolgende Beispiel dient dazu die Erfindung zu erläutern, ohne deren Umfang einzuschränken.

### Beispiel 1: Rezeptur für eine (cremige) Emulsion:

- Wirkstoffe:: 10 Gew.% ätherisches Öl vom Teebaum
1 Gew.% Ratanhiawurzel Extrakt Tinktur (1:2)
- Hilfsstoffe:: Mandelöl
Lanolin
Cetylalkohol
Emulgatoren, Stearinsäure und Triethanolamin
Wässrige Phase 69%

### Beispiel 2: Rezeptur für eine (cremige) Emulsion (Dermaphytonikum):

- Wirkstoffe:: 10 Gew.% ätherisches Öl vom Teebaum
0,05 Gew.% Ratanhiawurzel Extrakt Siccum (5:1)
- Hilfsstoffe:: Mandelöl
Lanolin
Cetylalkohol
Emulgatoren, Stearinsäure und Triethanolamin
Wässrige Phase 69%

### Beispiel 3: Rezeptur für eine (cremige) Emulsion:

- Wirkstoffe:: 10 Gew.% ätherisches Öl vom Majoran
1 Gew.% Ratanhiawurzel Extrakt Tinktur (1:2)
- Hilfsstoffe:: Mandelöl
Lanolin
Cetylalkohol
Emulgatoren, Stearinsäure und Triethanolamin
Wässrige Phase 69%

### Beispiel 4: Rezeptur für eine (cremige) Emulsion:

- Wirkstoffe:: 10 Gew.% ätherisches Öl vom Teebaum
1% Eichenrinde (Quercus cortex) in Extraktform
- Hilfsstoffe:: Mandelöl
Lanolin
Cetylalkohol
Emulgatoren, Stearinsäure und Triethanolamin
Wässrige Phase 69%

### Beispiel 5: Rezeptur für eine (cremige) Emulsion:

- Wirkstoffe:: 15 Gew.% ätherisches Öl vom Teebaum
2% Tormentillae radix in Extraktform
- Hilfsstoffe:: Mandelöl
Lanolin
Cetylalkohol
Emulgatoren, Stearinsäure und Triethanolamin
Wässrige Phase 69%

### Beispiel 6: Rezeptur für eine (cremige) Emulsion:

- Wirkstoffe:: 10 Gew.% ätherisches Öl vom Majoran
1 Gew.% Hamamelidis cortex in Extraktform
- Hilfsstoffe:: Mandelöl
Lanolin
Cetylalkohol
Emulgatoren, Stearinsäure und Triethanolamin
Wässrige Phase 69%

### Beispiel 7: Klinische Studie

### EINE OFFENE, MULTIZENTRISCHE STUDIE ZUR WIRKSAMKEIT UND SICHERHEIT VON DERMA-PHYTONIKUM CREME BEI HUNDEN UND KATZEN BEI DER BEHANDLUNG VON CHRONISCHER UND/ODER ALLERGISCHER DERMATITIS MIT ODER OHNE JUCKREIZ, DURCH VERGLEICH DES SCHWEREGRADES VOR UND NACH DER BEHANDLUNG.

Eine offene, multizentrische, kontrollierte klinische Studie wurde durchgeführt, um die Wirksamkeit und Sicherheit der DERMA-PHYTONIKUM Creme, welche in Beispiel 2 beschrieben ist, bei der Behandlung von chronischer und/oder allergischer Dermatitis bei Hunden und Katzen nachzuweisen. Die Studie wurde gemäß der 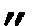 International Conference on Harmonization of Technical Requirements for Registration of Veterinary Medicinal Products (VICH), Topic GL 9" und der 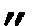 Guideline on Good Clinical Practices (December 1998)", durchgeführt.

Vierzehn (14) in der Schweiz niedergelassene Tierärzte wählten die Tiere für die klinische Studie aus. Dies erfolgte nach schriftlicher Einverständniserklärung der Tierbesitzer. Die Tiere mussten Ein- und Ausschlußkriterien erfüllen um an der Studie teilnehmen zu können. Diese Kriterien sind im Studienprotokoll festgehalten. Der (Die) Tierarzt(in) unterzog die Tiere bei ihrem ersten Besuch einer klinischen Untersuchung (Tabelle 1).

**Tabelle 1**

| Vorkommen von Dermatitis bei Hunden und Katzen | | |
|---|---|---|
| Diagnose der Dermatitis | Hunde (n=53) | Katzen (n=19) |
| Nicht-spezifisches Ekzem | 30 (56.6%) | 15 (78.9%) |
| Allergische Dermatitis | 15 (28.3%) | 3 (15.8%) |
| Allergische Flohdermatitis | 2 (3.8%) | 1 (5.3%) |
| Kontaktdermatitis | 3 (5.7%) | 3 (15.8%) |
| Eosinophiles Granulomkomplex bei der Katze | 0 | 2 (10.5%) |
| Zwischenzehen Pyodermie | 5 (9.4%) | 0 |
| Leckdermatitis | 21 (39.6%) | 5 (26.3%) |
| Zwischenfalten Pyodermie | 4 (7.5%) | 0 |

auftreten, sowie die Diagnose und Ursache der Dermatitis (falls bekannt), wurden ebenfalls in den Aufzeichnungen festgehalten. Der Halter des Haustiers trug DERMA-PHYTONIKUM zweimal täglich über einen Zeitraum von 28 Tagen dünn auf die Läsionen auf. Der Halter zeichnete dies in einem 'Tagebuch des Haustierhalters' auf und hielt dort auch seine Einschätzung über die Fortschritte in bezug auf den Zustand des Haustiers mit dem Urteil ,besser', ,unverändert' oder ,schlechter' fest. Der Tierbesitzer besuchte den Tierarzt zwei bis viermal während der 28-tägigen Behandlungsdauer. Der Tierarzt führte nach Ende der Behandlung eine abschließende Untersuchung durch. Dabei wurden alle unerwünschten Ereignisse aufgezeichnet. Folgende Medikamente wurden während der Studie ausgeschlossen: Corticosteroide, Antibiotika und Phytopharmaka.

Der Tierarzt überprüfte bei jeder Visite und nach 28 Behandlungstagen die Wirksamkeit des Präparates bei allen Tieren. Die Wirksamkeit wurde bestätigt, in dem die Aufzeichnungen über den Schweregrad der Symptome für Juckreiz, Erythem, Abschürfung, Papeln, Pusteln, Knoten, Eiter, Wundschorf, Alopezie, Hautverdickung, Urtikaria, Schuppigkeit, Überpigmentierung und Blasen, die vor und nach der Behandlung mit DERMA-PHYTONIKUM erfolgten, ausgewertet wurden.

Eine Gesamtbewertung der Wirksamkeit wurde vom Tierarzt nach Abschluß der Behandlung beim abschließenden Besuch durchgeführt und diese wurde mit dem Urteil 'sehr gut', 'gut', 'mittelmäßig', oder 'nicht zufriedenstellend' beurteilt.

Der Tierarzt bewertete die Gesamtverträglichkeit der Behandlung bei jedem Tier, indem die Häufigkeit des Auftretens von unerwünschten Nebenwirkungen und die Art der aufgetretenen unerwünschten Nebenwirkungen berücksichtigt wurden. Die Verträglichkeit wurde mit 'sehr gut', 'gut', 'mittelmäßig' oder 'nicht zufriedenstellend' beurteilt.

### Resultate der Wirksamkeit bei Hunden und Katzen

Zweiundsiebzig (72) Tiere, 53 Hunde und 19 Katzen wurden für die Studie registriert (Tabelle 1). Jedes Tier, das unter chronischen Bedingungen leidet, wird in der Studie eingeschlossen. In der 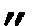 Steady State 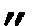 Situation wurde jedes Tier als seine eigene Kontrolle betrachtet. Drei Hunde und drei Katzen haben die Studie aufgrund des Auftretens von unerwünschten Wirkungen nicht abgeschlossen. Nur zwei Abbrüche konnten mit gewisser Wahrscheinlichkeit auf die Anwendung von DERMA-PHYTONIKUM, zurückgeführt werden. Für vier Hunde und eine Katze wurde die Studie aus anderen Gründen abgebrochen. Alle aufgezeichneten Daten wurden ausgewertet.

Die Wirksamkeits- und Verträglichkeitsdaten über alle Tiere wurden mit Hilfe von Version 6.12 des SAS-Statistikpakets analysiert. Die statistische Analyse der Gesamtwirksamkeit ergab, daß 82 % (42/51) der Hunde (für 2 Hunde war eine Bewertung nicht verfügbar), die an chronischer und/oder allergischer Dermatitis litten und die mit DERMA-PHYTONIKUM behandelt wurden, 'gut' oder 'sehr gut' auf die Behandlung ansprachen, daraus ergibt sich bei einer Signifikanzstufe von 5% mit 95%-iger Sicherheit ein Vertrauensbereich des Mittelwertes von 72% bis 92% (Tabelle 2).

Entsprechend wurde bei 84% (16/19) der Katzen eine ,gute' oder ,sehr gute' Gesamtwirksamkeit festgestellt, daraus ergibt sich bei einer Signifikanzstufe von 5% mit 95 %-iger Sicherheit ein Vertrauensbereich des Mittelwertes von 68% bis 100% (Tabelle 2).

**Tabelle 2**

| Beurteilung des Tierarztes über die Wirksamkeitsdaten bei Hunden und Katzen | | | |
|---|---|---|---|
| Spezies | Gesamtwirksamkeit nach Behandlung mit Derma-Phytonikum | | |
| | Sehr Gut/Gut | Mässig | Unbefriedigend |
| Hund (n=51)* | 42 (82%) | 4 (7.8%) | 5 (9.8%) |
| Katze (n=19) | 16 (84%) | 2 (10.5%) | 1 (5.3%) |

| | | | |
|---|---|---|---|
| *Original Prüfbericht nicht ergänzt für Gesamtwirksamkeit für 2 Hunde | | | |

Zwischen dem ersten Besuch, vor der Behandlung, und dem 4. Besuch nach 4-wöchiger Behandlung ist sowohl ein deutliche Abnahme der Symptomhäufigkeit, als auch eine statistisch signifikante Reduktion des Schweregrades der Symptome festzustellen.

Die Behandlung mit DERMA-PHYTONIKUM verbessert die Symptome Juckreiz, Erythem, Abschürfung, Pusteln, Knoten, Wundschorf, Alopezie, Hautverdickung und Schuppigkeit bei Hunden signifikant (Bowkers Statistik) (Tabellen 3 und 4).

**Tabelle 3**

| Behandlung mit Derma-Phytonikum: Symptome beim Hund vor der klinischen Behandlung, am Tag 15 (+/-3Tage) und nach 4 Wochen Behandlung | | | |
|---|---|---|---|
| Klinische Symptome der Dermatitis beim Hund | Besuch 1 vor der Behandlung n = 53 % | Besuch 2 während der Behandlung n = 30 % | Besuch 4 nach 4 Wochen Behandlung n = 47 % |
| Juckreiz | 96.2 | 88.6 | 27.7 |
| Erythem | 88.7 | 68.6 | 21.3 |
| Abschürfung | 58.5 | 45.7 | 6.4 |
| Pappeln | 20.8 | 14.3 | 2.1 |
| Pusteln | 30.2 | 20.0 | 2.1 |
| Knoten | 66.0 | 34.3 | 6.4 |
| Eiter | 35.8 | 17.1 | 2.1 |
| Wundschorf | 50.9 | 51.4 | 21.3 |
| Alopezie | 73.6 | 57.1 | 38.3 |
| Hautverdickung | 58.5 | 40.0 | 23.4 |
| Urtikaria | 5.7 | 0.00 | 2.1 |
| Schuppigkeit | 50.9 | 57.1 | 34.0 |
| Überpigmentierung | 18.9 | 11.4 | 19.1 |
| Blasen | 3.8 | 2.9 | 0.0 |
| n = Anzahl der Tiere | | | |

**Tabelle 4**

| Reduktion des Schweregrades der Symptome bei Hunde nach 4-wöchiger Behandlung mit Derma-Phytonikum | | |
|---|---|---|
| Klinische Symptome der Dermatitits beim Hund | Bowkers Statistic Besuch1: Besuch4 | |
| | Signifikanz | |
| | JA | NEIN |
| Juckreiz | 43.0 | |
| Erythem | 36.7 | |
| Abschürfung | 27.0 | |
| Pappeln | | 10.0 |
| Pusteln | 14.0 | |
| Knoten | 31.0 | |
| Eiter | 17.0 | |
| Wundschorf | 20.3 | |
| Alopezie | 31.0 | |
| Hautverdickung | 24.0 | |
| Urtikaria | | 2.0 |
| Schuppigkeit | 16.6 | |
| Überpigmentierung | | 2.5 |
| Blasen | | 2.0 |

Zwischen Besuch 1, vor der Behandlung, und Besuch 4 nach 4-wöchiger Behandlung ist sowohl eine deutliche Abnahme der Symptomhäufigkeit als auch eine statistisch signifikante Verbesserung, d.h. eine signifikante Reduktion des Schweregrades der Symptome Juckreiz und Wundschorf bei der Katze festzustellen (Tabellen 5 und 6).

**Tabelle 5**

| Behandlung mit Derma-Phytonikum: Symptome bei der Katze vor der Behandlung (Besuch 1), am Tag 15 (+/- 3Tage) (Besuch 2) und nach 4 Wochen Behandlung (Besuch 4) | | | |
|---|---|---|---|
| Klinische Symptome der Dermatitis bei der Katze | Besuch 1 Vor der Behandlung n = 19 % | Besuch 2 Während der Behandlung n = 12 % | Besuch 4 Nach 4 Wochen Behandlung n = 16 % |
| Juckreiz | 100.0 | 92.3 | 12.5 |
| Erythem | 68.4 | 76.9 | 18.8 |
| Abschürfung | 78.9 | 76.9 | 12.5 |
| Pappeln | 5.3 | 0.0 | 6.3 |
| Pusteln | 15.8 | 7.7 | 0.0 |
| Knoten | 68.4 | 23.1 | 12.5 |
| Eiter | 5.3 | 0.0 | 0.0 |
| Wundschorf | 94.7 | 76.9 | 25.0 |
| Alopezie | 94.7 | 53.8 | 43.8 |
| Hautverdickung | 21.1 | 15.4 | 6.3 |
| Urtikaria | 0.0 | 0.0 | 0.0 |
| Schuppigkeit | 63.2 | 53.8 | 12.5 |
| Überpigmentierung | 0.0 | 0.0 | 0.0 |
| Blasen | 5.3 | 0.0 | 0.0 |
| n = Anzahl der Tiere | | | |

**Tabelle 6**

| Reduktion des Schweregrades der Symptome bei Katzen nach 4-wöchiger Behandlung mitDerma-Phytonikum | | |
|---|---|---|
| Klinische Symptome der Dermatitis bei der Katze | Bowkers Statistik Besuch 1: Besuch 4 | |
| | Signifikanz | |
| | JA | NEIN |
| Juckreiz | 16.0 | |
| Erythem | | 11.0 |
| Abschürfung | | 11.0 |
| Pappeln | | 1.0 |
| Pusteln | | 3.0 |
| Knoten | | 8.8 |
| Eiter | | 1.0 |
| Wundschorf | 14.0 | |
| Alopezie | | 11.7 |
| Hautverdickung | | 2.3 |
| Urtikaria | - | - |
| Schuppigkeit | | 10.0 |
| Überpigmentierung | - | - |
| Blasen | | 1.0 |

### Resultate der Verträglichkeit

Die Verträglichkeit wurde primär auf der Grundlage des Auftretens von unerwünschten Wirkungen und der Art der unerwünschten Wirkungen bewertet.

Bei 85% (44/52) der Hunde konnte eine ,gute' oder ,sehr gute' Gesamtverträglichkeit festgestellt werden, daraus ergibt sich bei einem Vertrauensinterval von 5% mit 95%-iger Sicherheit ein Vertrauensbereich des Mittelwertes von 75% bis 95% (Tabelle 7).

Bei 84% (16/19) der Katzen konnte eine ,gute' oder ,sehr gute' Gesamtverträglichkeit festgestellt werden, daraus ergibt sich bei einem Vertrauensinterval von 5% mit 95%-iger Sicherheit ein Vertrauensbereich des Mittelwertes von 68% bis 100% (Tabelle 7).

**Tabelle 7**

| Beurteilung der Verträglichkeit bei Hunden und Katzen durch den Tierarzt | | | |
|---|---|---|---|
| Spezies | Gesamtverträglichkeit nach Behandlung mit Derma-Phytonikum | | |
| | Sehr Gut/Gut | Mässig | Unbefriedigend |
| Hund (n=52)* | 44 (85%) | 7 (13.5%) | 1 (1.9%) |
| Katze (n=19) | 16 (84%) | 2 (10.5%) | 1 (5.3%) |

| | | | |
|---|---|---|---|
| *Original CRF nicht ergänzt für Gesamtverträglichkeit für 1 Hund | | | |

Die Auswertung ergab, daß nur für zwei Hunde (2/53) unerwünschte Ereignisse dokumentiert wurden, die eventuell auf die Anwendung von DERMA-PHYTONIKUM zurückzuführen waren. Bei beiden Hunden wurden nach interdigitaler Anwendung vorübergehend lokalisierte Hautirritationen und Hyperämie beobachtet.

Für drei Katzen (3/19) wurden unerwünschte Ereignisse bei der Anwendung von DERMA-PHYTONIKUM beschrieben. Bei allen drei Katzen erfolgte die Anwendung im Ohr (zwischen dem Auge und dem äusseren Ohreingang), dabei traten lokale, vorübergehende Reaktionen an der Behandlungsstelle auf.

### Allgemeine Schlußfolgerungen über die klinische Dokumentation von Derma-Phytonikum

Durch den klinisch kontrollierten Einsatzversuch bei Tierärzten in der Schweiz konnte die positive Wirksamkeit und Verträglichkeit von DERMA-PHYTONIKUM bei der Behandlung von chronischer und/oder allergischer Dermatitis bei zweimal täglicher topischer Anwendung über einen Zeitraum von höchstens 28 aufeinanderfolgenden Tagen bei Hunden und Katzen klar nachgewiesen werden.

## Patentansprüche

1. Eine therapeutisch wirksame wässrige Kombination enthaltend 5 bis 25 Gew.% von Terpinen-4-ol enthaltenden ätherischen Ölen und 0.01 - 10 Gew.% von Gerbstoffe enthaltenden Arzneipflanzen bzw. deren Extrakte oder Tinkturen, zur topischen Behandlung von Haut- und Schleimhaut Affektionen.

2. Therapeutisch wirksame Kombination gemäss Patentanspruch 1 zur topischen Behandlung der Haut- und Schleimhaut von Menschen.

3. Therapeutisch wirksame Kombination gemäss Patentanspruch 1 zur topischen Behandlung der Haut- und Schleimhaut von Tieren.

4. Therapeutisch wirksame Kombination gemäss einem der vorangegangenen Ansprüche, enthaltend 10 Gew.% von Terpinen-4-ol enthaltenden ätherischen Ölen.

5. Therapeutisch wirksame Kombination gemäss einem der vorangegangenen Ansprüche, wobei der Extrakt bzw. die Tinktur der Gerbstoffe enthaltenden Arzneipflanzen 0.1-2 Gew.% der Droge enthält.

6. Therapeutisch wirksame Kombination gemäss einem der vorangegangenen Ansprüche dadurch gekennzeichnet, dass das ätherische Öl mindestens 20% Terpinen-4-ol enthält.

7. Therapeutisch wirksame Kombination gemäss einem der vorangegangenen Ansprüche, wobei das Terpinen-4-ol enthaltende ätherische Öl Teebaumöl ist.

8. Therapeutisch wirksame Kombination gemäss einem der vorangegangenen Ansprüche dadurch gekennzeichnet, dass in den Gerbstoffe enthaltenen Arzneipflanzen mindestens 2 Gew.% Gerbstoffe vorhanden sind.

9. Therapeutisch wirksame Kombination gemäss einem der vorangegangenen Ansprüche dadurch gekennzeichnet, dass die Gerbstoff enthaltende Arzneipflanze bzw. deren Extrakt ein Ratanhiawurzel-Extrakt ist.

10. Therapeutisch wirksame Kombination gemäss einem der vorangegangenen Ansprüche, dadurch gekennzeichnet, dass die neue, therapeutisch wirksame Kombination bei der Behandlung von chronischer Dermatitis unspezifischen Ekzemen, bei allergischer Dermatitis, bei Flohbissallergie, bei der Zwischenzehenpyodermie, Leckdermatitis und der Hautfaltenpyodermie von Hund und Katze, aber auch von Schnitt- und Schürfwunden, Bakterien- und Pilzinfektionen, von Infektionen im Urogenitalbereich, Geschwüren und Furunkeln, ferner aber auch von Allergien und anderen Hautproblemen, oder auch von Mischinfektionen der vorgenannten Art wirksam ist.

11. Therapeutisch wirksame Kombination gemäss einem der vorangegangenen Ansprüche, dadurch gekennzeichnet, dass sie in einer topischen Anwendungsform als Emulsion vorliegt.

12. Therapeutisch wirksame Kombination gemäss einem der vorangegangenen Ansprüche, dadurch gekennzeichnet, dass sie in einer topischen Anwendungsform, beispielsweise als Creme, Salbe, Gel oder in flüssiger Form als Öl oder Tinktur vorliegt.

13. Derma-Phytonikum, enthaltend eine Kombination gemäss einem der vorangegangenen Ansprüche zur topischen Behandlung von Haut- und Schleimhaut Affektionen im Tiergesundheitsbereich.

14. Mittel zur topischen Behandlung von Haut und Schleimhaut Affektionen enthaltend eine therapeutisch wirksame Kombination gemäss einem der vorangegangenen Ansprüche.

15. Verwendung einer Kombination gemäss einem der vorangegangenen Ansprüche zur Behandlung von Haut und Schleimhaut Affektionen.
